# EUROPÄISCHE PATENTSCHRIFT

(11) **EP 3 033 998 B1**
(45) Veröffentlichungstag und Bekanntmachung des Hinweises auf die Patenterteilung: **12.02.2020**
(21) Anmeldenummer: 15200215.0
(22) Anmeldetag: 15.12.2015
(51) Int. Cl.: A61B 5/145, A61M 25/00, B01D 63/06

(54) **MEHRLUMEN-MIKROKATHETERSCHLAUCH SOWIE VERFAHREN ZUM HERSTELLEN EINES MEHRLUMEN-MIKROKATHETERSCHLAUCHS**
MULTI-LUMEN MICRO-CATHETER HOSE AND METHOD FOR PRODUCING A MULTI-LUMEN MICRO-CATHETER HOSE
TUYAU DE MICRO-CATHETER A LUMIERES MULTIPLES ET PROCEDE DE FABRICATION D'UN TUYAU DE MICRO-CATHETER A LUMIERES MULTIPLES

(30) Priorität: 19.12.2014 DE 102014226628
(43) Veröffentlichungstag der Anmeldung: 22.06.2016
(73) Patentinhaber: RAUMEDIC AG, 95213 Münchberg (DE)
(72) Erfinder: Gläsel, Björn, 95158 Kirchenlamitz (DE)
(74) Vertreter: Rau, Schneck & Hübner Patentanwälte Rechtsanwälte PartGmbB

(56) Entgegenhaltungen:
- EP-A1- 2 027 812
- WO-A1-01/03752
- WO-A1-2009/095020
- WO-A2-2004/060465
- DE-C1- 19 714 572
- US-A1- 2005 137 579

## Beschreibung

Die Erfindung betrifft einen Mehrlumen-Mikrokatheterschlauch mit mindestens zwei Schlauchlumen. Ferner betrifft die Erfindung ein Verfahren zur Herstellung eines derartigen Mehrlumen-Mikrokatheterschlauchs.

Mehrlumenschläuche sind bekannt aus der WO 2014/149 708 A1, der WO 2009/095020 A1, der DE 197 14 572 C1, der WO 01/03752 A1, der EP 2 027 812 A1, der WO 2004/060465 A2 und der US 2005/0137579 A1. Derartige Mehrlumenschläuche können in der Medizintechnik als Mikrokatheterschläuche zum Einsatz kommen.

Es ist eine Aufgabe der vorliegenden Erfindung, einen Mehrlumenschlauch derart weiterzubilden, dass dessen Einsatzmöglichkeiten beispielsweise als Sonde zum Messen von Gewebeparametern verbessert sind.

Diese Aufgabe ist erfindungsgemäß gelöst durch einen Mehrlumen-Mikrokatheterschlauch mit den im Anspruch 1 angegebenen Merkmalen.

Der erfindungsgemäße Mehrlumen-Mikrokatheterschlauch ermöglicht es, ein Medium über eines der Schlauchlumen hin zum distalen Schlauchende und über die mindestens eine Durchtrittsöffnung und das andere der beiden Schlauchlumen vom distalen Schlauchende wieder zurückzuführen. Hierdurch kann ein Diagnose-, Analyse- oder Therapiemedium hin zu einem Einsatzort im Bereich des distalen Schlauchendes geführt werden und kann dort ggf. gezielt mit Gewebematerial wechselwirken. Ein proximales Schlauchende kann mit einer Zuführ- bzw. Abführeinrichtung zum Zu- bzw. Abführen eines Mediums in Fluidverbindung stehen.

Mindestens eine Fenster-Durchtrittsöffnung in mindestens einem der Schlauchlumen, wobei über die mindestens eine Fenster-Durchtrittsöffnung ein Medienaustausch zwischen dem Schlauchlumen und einer Umgebung ermöglicht ist, gewährleistet eine intensive Austauschwechselwirkung zwischen einem inneren mindestens eines der Schlauchlumen und einer Schlauchumgebung. Die ggf. mehreren Fenster-Durchtrittsöffnungen können als Messfenster ausgeführt sein. Jeweils mindestens eine Fenster-Durchtrittsöffnung kann in beiden Schlauchlumen vorhanden sein. In diesem Fall können die Fenster-Durchtrittsöffnungen der beiden Schlauchlumen in Richtung einer Schlauch-Längsachse axial gegeneinander versetzt sein, was eine mechanische Stabilität des Mikrokatheterschlauchs erhöhen kann. Die mindestens eine Fenster-Durchtrittsöffnung kann nahe dem distalen Schlauchende ausgeführt sein. Der Mehrlumen-Mikrokatheterschlauch kann Innenlumen mit Innendurchmessern von 1 mm und darunter aufweisen.

Ein im Bereich der Fenster-Durchtrittsöffnung in das Schlauchlumen eingesetztes permeables Material ermöglicht einen definierten Medienaustausch zwischen einem über die Schlauchlumen zu- und abgeführten Medium und einer Umgebung des Mikrokatheterschlauchs. Das permeable Material ist für das im Mikrokatheterschlauch zu führende Medium permeabel. Alternativ oder zusätzlich kann das permeable Material für den Mikrokatheterschlauch im Bereich der Fenster-Durchtrittsöffnung umgebendes Gewebematerial oder Blut permeabel sein. Bei dem permeablen Material handelt es sich um ein Fasermaterial. Das permeable Material ist in Richtung der Schlauch-Längsachse, also axial, länger als die zugehörige Fenster-Durchtrittsöffnung. Hierdurch ist eine axiale Fixierung des permeablen Materials im zugehörigen Schlauchlumen gewährleistet.

Genau eine Fenster-Durchtrittsöffnung nach Anspruch 3 verringert einen Herstellungsaufwand und ermöglicht reproduzierbare Bedingungen für einen Medienaustausch. Alternativ können mehrere Fenster-Durchtrittsöffnungen pro Schlauchlumen zum Einsatz kommen. Soweit eines der Schlauchlumen genau eine Fenster-Durchtrittsöffnung aufweist, kann dies für alle Schlauchlumen gelten. Alternativ kann eines der Schlauchlumen genau eine Fenster-Durchtrittsöffnung aufweisen und ein anderes Schlauchlumen mehrere Fenster-Durchtrittsöffnungen aufweisen.

Eine Klebstoffschicht nach Anspruch 4 sorgt für eine sichere Fixierung des permeablen Materials. Zudem kann die Klebstoffschicht zu einer Abdichtung des permeablen Materials hin zu einer Innenwand des zugehörigen Schlauchlumens führen. Dies erzwingt einen Durchtritt des zu- bzw. abgeführten Mediums durch das permeable Material und vermeidet einen Kurzschlussstrom zwischen dem Schlauchlumen und der Mikrokatheterschlauch-Umgebung am permeablen Material vorbei. Die Klebstoffschicht kann durch einen gezielt aushärtbaren Klebstoff gebildet sein. Eine Viskosität des Klebstoffs kann vor dem Aushärten im Bereich zwischen 250 und 400 Pa s liegen.

Ein fluiddichter Verschluss des distalen Schlauchendes nach Anspruch 5 vermeidet einen unerwünschten Medienaustritt am distalen Ende des Mikrokatheterschlauchs. Alternativ kann auch das distale Schlauchende durch ein permeables Material verschlossen sein, sodass auch eine distale Öffnung des Mikrokatheterschlauchs als Messfenster genutzt werden kann. Ein derartiger Verschluss durch permeables Material kann durch einen Membran-Verschlussstopfen oder durch einen Verschlussstopfen aus Fasermaterial realisiert sein.

Innendurchmesser der Schlauchlumen nach Anspruch 6 haben sich für den jeweiligen Anwendungszweck für besonders geeignet herausgestellt. Die Schlauchlumen können den gleichen Innendurchmesser haben. Dies ist allerdings nicht zwingend.

Ein Herstellungsverfahren nach Anspruch 7 kann mit Massenproduktionsmethoden realisiert sein. Der Mehrlumenschlauch kann durch Extrusion hergestellt sein. Die Fenster-Durchtrittsöffnung kann durch Stanzen oder Schneiden angebracht werden. Beim Verkleben des permeablen Materials im Schlauchlumen kann ein Kapillareffekt des Klebstoffs ausgenutzt werden. Hierzu kann eine Viskosität oder eine Menge des Klebstoffs auf eine Dimensionierung eines Zwischenraums zwischen einer Schlauchlumen-Wandung und dem permeablen Material im Bereich der jeweiligen Fenster-Durchtrittsöffnung abgestimmt sein.

Ein Herstellungsverfahren nach Anspruch 8 ermöglicht eine Ausbildung einer Durchtrittsöffnung zwischen dem verbleibenden Trennsteg und dem Verschluss des distalen Schlauchendes und ermöglicht damit einen Interlumen-Übergang zwischen den Schlauchlumen des Mehrlumenschlauchs, wobei auch dieses Herstellungsverfahren mit Methoden der Massenproduktion realisiert sein kann.

Ein nach Anspruch 9 verschließender Klebstoff kann eine Viskosität im Bereich zwischen 400 und 800 Pa s haben. Beim verklebenden Verschließen des distalen Schlauchlumens kann ein Kapillareffekt des Klebstoffs ausgenutzt werden, entsprechend dem, was vorstehend im Zusammenhang mit dem Verkleben des permeablen Materials bereits ausgeführt wurde. Die Mikrokatheterschlauch-Herstellungsverfahren, die vorstehend erläutert wurden, können miteinander kombiniert werden.

Der Klebstoff kann jeweils über eine entsprechende Dosiereinrichtung an der jeweiligen Klebeposition zudosiert werden. Eine solche Dosiereinrichtung kann als Spritze ausgeführt sein.

Ausführungsbeispiele der Erfindung werden nachfolgend anhand der Zeichnungen näher erläutert. In dieser zeigen:
- Fig. 1: einen Querschnitt eines Mehrlumen-Mikrokatheterschlauchs am Beispiel eines Zweilumenschlauchs;
- Fig. 2: einen axialen Längsschnitt des Mikrokatheterschlauchs nach Fig. 1 im Bereich eines distalen Schlauchendes;
- Fig. 3: eine Stirnansicht auf den Mikrokatheterschlauch aus Blickrichtung III in Fig. 2;
- Fig. 4: in einer zur Fig. 2 ähnlichen Darstellung den Mikrokatheterschlauch mit mittels einem Klebstoff-Verschlussstopfen verschlossenen distalen Schlauchende sowie in die beiden Schlauchlumen eingesetztem Fasermaterial;
- Fig. 5: in einem axialen Längsschnitt einen Abschnitt des Mikrokatheterschlauchs im Bereich von Fenster-Durchtrittsöffnungen, über die ein Medienaustausch zwischen den Schlauchlumen und einer Umgebung ermöglicht ist;
- Fig. 6: in einer zur Fig. 5 ähnlichen Darstellung den Schlauchabschnitt nach dem Einsetzen von Fasermaterial in die Schlauchlumen auf Höhe der jeweiligen Fenster-Durchtrittsöffnung;
- Fig. 7: in einer zur Fig. 6 ähnlichen Darstellung den Schlauchabschnitt nach dem Fixieren des Fasermaterials in den Schlauchlumen durch Verkleben;
- Fig. 8 bis 10: in einer zur Fig. 5 ähnlichen Darstellung den Schlauchabschnitt einschließlich des distalen Schlauchendes mit weiteren Varianten eingebrachter Fenster-Durchtrittsöffnungen;
- Fig. 11: eine Seitenansicht des Schlauchabschnitts nach Fig. 10 aus Blickrichtung XI in Fig. 10;
- Fig. 12: in einer zur Fig. 4 ähnlichen Darstellung den Mikrokatheterschlauch mit einer weiteren Variante angebrachter Fenster-Durchtrittsöffnungen, wobei gegenüberliegend dem distalen Schlauchende ein Verbindungsadapter zu einer nicht dargestellten Zuführ- bzw. Abführeinrichtung eines Kathetersystems angedeutet ist.

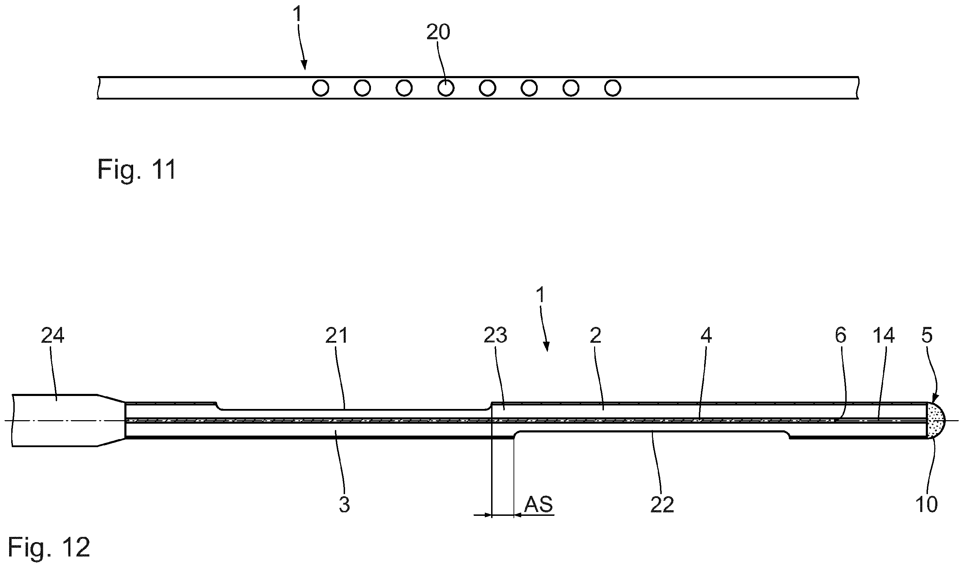

Ein in der Fig. 12 dargestellter Mehrlumen-Mikrokatheterschlauch 1 kann als Bestandteil einer Dialysesonde zum Einsatz kommen. Ein derartiges Anwendungsgebiet ist beschrieben in der DE 693 23 563 T2.

Der Mikrokatheterschlauch 1 ist als Zweilumenschlauch ausgeführt. Der Mikrokatheterschlauch 1 ist aus einem verklebbaren Kohlenstoff, beispielsweise aus einem thermoplastischen Kohlenstoff. Beispiele hierfür sind Polyetherimid (PEI), Polyether-block-amid (PEBA), Polyamid (PA) oder Polysulfon (PSU). Auch Copolymere aus diesen Materialien können zum Einsatz kommen.

Der Mikrokatheterschlauch 1 hat zwei Schlauchlumen 2, 3. Die Schlauchlumen 2, 3 haben einen Innendurchmesser im Bereich zwischen 0,1 mm und 2 mm. Beim dargestellten Beispiel beträgt der Innendurchmesser etwa 0,3 mm. Ein Trennsteg 4 zwischen den beiden Schlauchlumen 2, 3 hat eine minimale Stärke von etwa 0,1 mm. Auch geringere minimale Stärken sind möglich.

Nicht dargestellte Ausführungen des Mikrokatheterschlauchs 1 können auch mehr als zwei Schlauchlumen aufweisen. Die Innendurchmesser der Schlauchlumen 2, 3 sind bei der dargestellten Ausführung gleich groß. Auch unterschiedliche Innendurchmesser sind möglich.

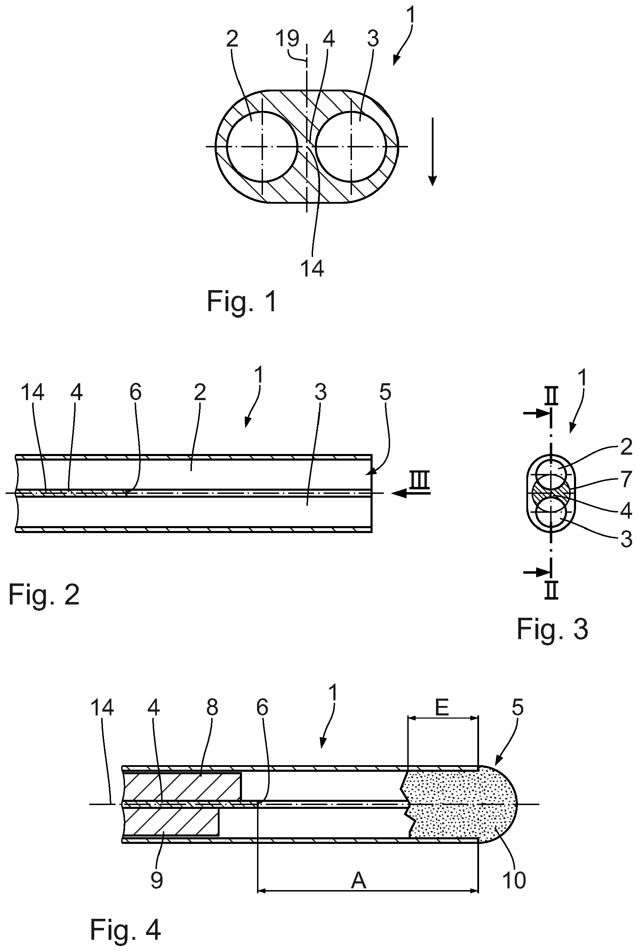

Die Figuren 2 und 3 zeigen den Mikrokatheterschlauch 1 im Bereich eines distalen Schlauchendes 5. In diesem Bereich ist der Trennsteg 4 entfernt, sodass eine Interlumen-Durchtrittsöffnung 6 resultiert, die einen Mediendurchtritt zwischen den Schlauchlumen 2 und 3 ermöglicht. Die Entfernung des Trennstegs 4 im Bereich des distalen Schlauchendes 5 kann durch Materialabtrag von der distalen Stirnseite des Mikrokatheterschlauchs 1 her erfolgen. Dies ist in der Figur 3 durch Andeutung eines kreisförmigen und den Trennsteg 4 überdeckenden Materialabtragbereiches 7 angedeutet.

Figur 4 zeigt den Mikrokatheterschlauch 1 wiederum im Bereich des distalen Schlauchendes 5 nach der Durchführung weiterer Herstellungsschritte. In die Schlauchlumen 2 und 3 ist als permeables Material jeweils Fasermaterial in Form von Messfasern 8, 9 eingesetzt. Zudem ist das distale Schlauchende 5 fluiddicht verschlossen durch einen Klebstoff-Verschlussstopfen 10. Letzterer hat eine Eindringtiefe E in das distale Schlauchende 5, die so gering ist, dass die Interlumen-Durchtrittsöffnung 6 nicht durch den Klebstoff-Verschlussstopfen 10 verschlossen ist. Eine Abtragtiefe A des Trennstegs 4 ist also größer als die Eindringtiefe E. Nach dem Aushärten des Klebstoffs schließt der Klebstoff-Verschlussstopfen 10 das distale Ende 5 derart ab, dass im Bereich des Klebstoff-Verschluss-stopfens 10 ein Außendurchmesser des Mikrokatheterschlauchs 1 nicht vergrößert ist.

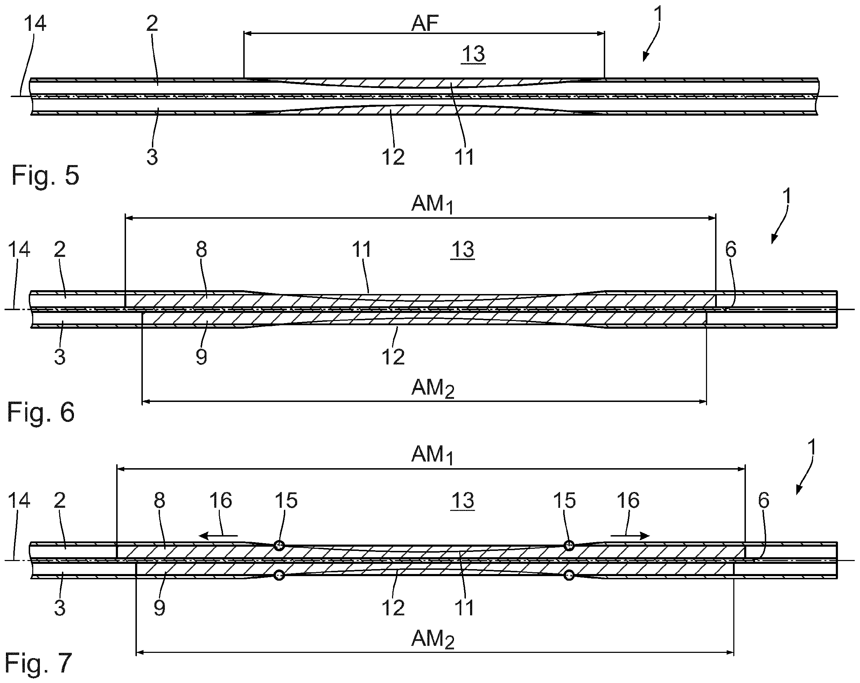

Figur 5 zeigt einen Schlauchabschnitt des Mikrokatheterschlauchs 1. Dieser Schlauchabschnitt kann im Bereich des distalen Schlauchendes vorliegen; dies ist aber nicht zwingend. Die beiden Schlauchlumen 2, 3 haben im Bereich dieses Schlauchabschnitts jeweils eine Fenster-Durchtrittsöffnung 11, 12, über die ein Medienaustausch zwischen den Schlauchlumen 2, 3 und einer Umgebung 13 um den Mikrokatheterschlauch 1 ermöglicht ist. Bei der Ausführung nach Fig. 5 weist jedes der Schlauchlumen 2, 3 genau eine Fenster-Durchtrittsöffnung 11, 12 auf.

Bei einer alternativen, nicht dargestellten Ausführung hat der Mikrokatheterschlauch 1 mindestens eine Fenster-Durchtrittsöffnung ausschließlich in einem der beiden Schlauchlumen 2 oder 3.

Eine Einbringung der Fenster-Durchtrittsöffnungen 11, 12 kann durch Stanzen oder Schneiden erfolgen.

Eine Axialerstreckung AF der Fenster-Durchtrittsöffnungen 11, 12, also eine Erstreckung längs einer Schlauch-Längsachse 14, liegt im Bereich zwischen 2 mm und 30 mm, besonders im Bereich zwischen 5 mm und 25 mm und kann beispielsweise 15 mm betragen.

Die Figuren 6 und 7 zeigen verschiedene Herstellschritte bei der weiteren Konfektionierung des Mikrokatheterschlauchs 1.

Bei der Momentandarstellung nach Fig. 6 sind die Messfasern 8 und 9 in die Schlauchlumen 2, 3 jeweils auf Höhe der Fenster-Durchtrittsöffnungen 11, 12 eingesetzt. Axialerstreckungen AM₁, AM₂ der Messfasern 8, 9 sind größer als die Axialersteckung AF der Messfenster 11, 12. Die Axialerstreckungen AM₁, AM₂ können sich voneinander unterscheiden; dies ist aber nicht zwingend. Da die Axialerstreckungen AM₁, AM₂ größer sind als die Axialerstreckung AF, kann eine Einsatzposition der Messfasern 8, 9 in die Schlauchlumen 2, 3 gewährleistet sein, bei der die Messfasern 8, 9 endseitig in die öffnungsfreien Lumenbereiche der Schlauchlumen 2, 3 hineinragen, was eine axiale Fixierung der Messfasern 8, 9 in den Schlauchlumen 2, 3 im Bereich der Fenster-Durchtrittsöffnungen 11, 12 begünstigt.

Fig. 7 zeigt den nachgelagerten Herstellschritt beim weiteren Fixieren der Messfasern 8, 9 in den Schlauchlumen 2, 3 durch Verkleben. Hierzu wird an Klebepositionen 15 ein zunächst flüssiger, aushärtbarer Klebstoff aufgebracht, dessen Viskosität und Menge abgestimmt ist auf ein Maß eines Klebespaltes zwischen einer inneren Schlauchlumen-Wandung und den Messfasern 8, 9. Dieser Klebespalt hat, gesehen in einem Schnitt senkrecht zur Schlauch-Längsachse 14, eine Spaltfläche im Bereich zwischen 0,01 mm² und 0,1 mm², beispielsweise eine Spaltfläche von 0,04 mm². Aufgrund der angesprochnen Viskositätsabstimmung wird der Klebstoff durch Kapillarwirkung in Richtung der Pfeile 16 in Fig. 7 in die an die Fenster-Durchtrittsöffnungen 11, 12 angrenzenden, öffnungsfreien Bereiche der Schlauchlumen 2, 3 eingezogen und umgibt die Messfasern 8, 9 anschließend vollumfänglich, also ringförmig, sodass die Messfasern 8, 9 mantelseitig fluiddicht gegen die jeweilige Innenwand der Schlauchlumen 2, 3 abgedichtet sind. Die Menge des Klebstoffs ist so abgestimmt, dass der Klebstoff nicht bis zu den stirnseitigen Enden der Messfasern 8, 9 vordringt, sodass ein unerwünschtes Verschließen dieser stirnseitigen Messfaser-Enden durch den Klebstoff vermieden ist.

Bei dem Klebstoff handelt es sich um einen gezielt aushärtbaren Klebstoff, beispielsweise um einen unter UV-Strahlung aushärtbaren Klebstoff, insbesondere auf Cyanacrylatbasis. Der Klebstoff kann eine Viskosität im Bereich von 2500 bis 4000 cP, also im Bereich zwischen 250 und 400 Pa s aufweisen. Alternativ oder zusätzlich zu einer UV-Aushärtung kann auch ein Klebstoff zum Einsatz kommen, bei dem eine Aushärtung über andere Einflussparameter erfolgt, beispielsweise über ein temperaturgesteuertes Aushärten, über ein luftfeuchtigkeitsgesteuertes Aushärten oder auch über ein rein zeitgesteuertes Aushärten.

Zum Verschließen des distalen Schlauchendes 5 mit Hilfe des Klebstoff-Verschlussstopfens 10 kommt ein ebenfalls hinsichtlich seiner Menge und Viskosität abgestimmter Klebstoff zum Einsatz, der dort insbesondere auf den Innendurchmesser des Mikrokatheterschlauchs 1 im Bereich des distalen Schlauchendes 5 abgestimmt ist. Dort ist der Innendurchmesser im Vergleich zum Innendurchmesser der Schlauchlumen 2, 3 aufgrund des Entfernens des Trennstegs 4 vergrößert und ist senkrecht zum Trennsteg 4 mehr als doppelt so groß als der Innendurchmesser der einzelnen Schlauchlumen 2, 3. Die Viskosität des Klebstoffs für den Klebstoff-Verschlussstopfen 10 wird so abgestimmt, dass die Eindringtiefe E resultiert, die kleiner ist als die Tiefe A des entfernten Trennstegs 4. Der Klebstoff wird am distalen Schlauchende 5 über eine Fläche aufgetragen, die eine Ausdehnung im Bereich zwischen 0,1 mm² und 0,5 mm² hat und beispielsweise bei 0,20 mm² liegen kann. Eine Viskosität des Klebstoffs für den Klebstoff-Verschlussstopfen 10 liegt im Bereich zwischen 4000 und 8000 cP, also im Bereich zwischen 400 und 800 Pa s. Zum Verkleben der Messfasern 8, 9 kommt also insbesondere ein anderer Klebstoff zum Einsatz als zur Herstellung des Klebstoff-Verschlussstopfens 10.

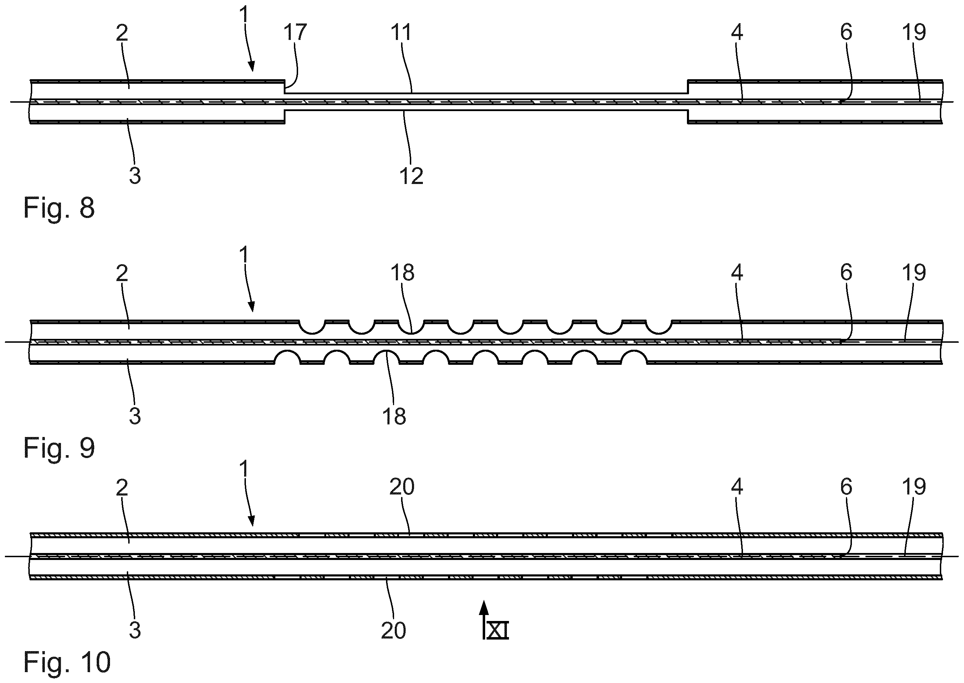

Die Fig. 8 bis 12 zeigen weitere Varianten von Fenster-Durchtrittsöffnungen, die anstelle der Fenster-Durchtrittsöffnungen 11, 12 zum Einsatz kommen können. Komponenten, die denjenigen entsprechen, die vorstehend in Bezugnahme auf die Fig. 1 bis 7 bereits erläutert wurden, tragen die gleichen Bezugsziffern und werden nicht nochmals im Einzelnen diskutiert.

Beim Mikrokatheterschlauch nach Fig. 8 sind die Fenster-Durchtrittsöffnungen 11, 12 als Stanzungen mit scharfen, axialendseitigen Stufen 17 ausgeführt.

Beim Mikrokatheterschlauch 1 nach Fig. 9 hat jedes der Schlauchlumen 2, 3 eine Mehrzahl, nämlich beispielhaft gezeigt jeweils acht, Fenster-Durchtrittsöffnungen 18. Diese sind als randseitige Stanzungen in den Außenwänden der Schlauchlumen 2, 3 ausgeführt, wobei eine Stanzbewegung parallel zu einer durch den Verlauf des Trennstegs 4 definierten Trennebene 19 (vgl. Fig. 1 und 9), also senkrecht zur Zeichenebene der Fig. 9, verläuft.

Bei der Ausführung des Mikrokatheterschlauchs 1 und nach den Fig. 10 und 11 sind Fenster-Durchtrittsöffnungen 20 durch Schneiden bzw. Stanzen mit einer Schneidbewegung senkrecht zur Trennebene 19 erzeugt.

Figur 12 zeigt eine Variante des Mikrokatheterschlauchs 1 mit zwei axial gegeneinander versetzten Fenster-Durchtrittsöffnungen 21, 22, die hinsichtlich ihrer Axialerstreckung den Fenster-Durchtrittsöffnungen 11, 12 nach den Fig. 5 und 8 entsprechen. Der Axialversatz der Fenster-Durchtrittsöffnungen 21, 22 zueinander ist so groß, dass die Fenster-Durchtrittsöffnungen 21, 22 axial nicht überlappen. Es verbleibt also in Axialrichtung zwischen den Fenster-Durchtrittsöffnungen 21, 22 ein Schlauchring 23 mit Axialerstreckung AS. Ein derartiger Axialversatz der Fenster-Durchtrittsöffnungen 21, 22 vergrößert die mechanische Stabilität des Mikrokatheterschlauchs 1.

Zusätzlich ist in der Fig. 12 an dem dem distalen Schlauchende 5 gegenüberliegenden Endbereich des Mikrokatheterschlauchs 1 ein Verbindungsadapter 24 abschnittsweise angedeutet. Der Verbindungsadapter 24 ist also am proximalen Schlauchende des Mikrokatheterschlauchs 1 angebracht. Über den Verbindungsadapter 24 kann dieses proximale Schlauchende mit einer Zuführ- bzw. Abführeinrichtung zum Zu- bzw. Abführen eines Mediums in Fluidverbindung stehen.

## Patentansprüche

1. Mehrlumen-Mikrokatheterschlauch (1)
- mit mindestens zwei Schlauchlumen (2, 3)
- mit einem verschlossenen distalen Schlauchende (5),
- wobei im Bereich des distalen Schlauchendes (5) ein Trennsteg (4) zwischen den beiden Schlauchlumen (2, 3) mindestens eine Durchtrittsöffnung (6) aufweist,
- wobei mindestens eines der Schlauchlumen (2, 3) mindestens eine Fenster-Durchtrittsöffnung (11, 12; 18; 20; 21, 22) aufweist, über die ein Medienaustausch zwischen dem Schlauchlumen (2, 3) und einer Umgebung (13) ermöglicht ist,
- wobei im Bereich der Fenster-Durchtrittsöffnung (11, 12; 18; 20; 21, 22) ein permeables Material in das Schlauchlumen (2, 3) eingesetzt ist,
- **dadurch gekennzeichnet, dass** das permeable Material als Fasermaterial in Form einer Messfaser (8, 9) in das Schlauchlumen (2, 3) eingesetzt ist,
- wobei die Messfaser (8, 9) in Richtung einer Schlauch-Längsachse (14), also axial, länger ist als die zugehörige Fenster-Durchtrittsöffnung (11, 12; 18; 20; 21, 22).

2. Mikrokatheterschlauch nach Anspruch 1, **dadurch gekennzeichnet, dass** mindestens eines der Schlauchlumen (2, 3) eine Mehrzahl von Fenster-Durchtrittsöffnungen (18; 20) aufweist.

3. Mikrokatheterschlauch nach Anspruch 1 oder 2, **dadurch gekennzeichnet, dass** mindestens eines der Schlauchlumen (2, 3) genau eine Fenster-Durchtrittsöffnung (11, 12; 21, 22) aufweist.

4. Mikrokatheterschlauch nach einem der Ansprüche 1 bis 3, **gekennzeichnet durch** eine Klebstoffschicht zum Fixieren der Messfaser (8, 9) im Schlauchlumen (2, 3).

5. Mikrokatheterschlauch nach einem der Ansprüche 1 bis 4, **dadurch gekennzeichnet, dass** das distale Schlauchende (5) fluiddicht verschlossen ist.

6. Mikrokatheterschlauch nach einem der Ansprüche 1 bis 5, **dadurch gekennzeichnet, dass** die Schlauchlumen (2, 3) einen Innendurchmesser im Bereich zwischen 0,1 mm und 2 mm haben.

7. Verfahren zum Herstellen eines Mehrlumen-Mikrokatheterschlauchs nach einem der Ansprüche 1 bis 6 mit folgenden Schritten:
- Herstellen eines Mehrlumenschlauchs,
- Einbringen der mindestens einen Fenster-Durchtrittsöffnung (11, 12; 18; 20; 21, 22) in mindestens eines der Schlauchlumen (2, 3),
- Einsetzen des permeablen Materials in das die Fenster-Durchtrittsöffnung (11, 12; 18; 20; 21, 22) aufweisende Schlauchlumen (2, 3) auf Höhe der Fenster-Durchtrittsöffnung (11, 12; 18; 20; 21, 22),
- Fixieren des permeablen Materials im Schlauchlumen (2, 3) durch Verkleben,
- **dadurch gekennzeichnet, dass** das permeable Material als Fasermaterial in Form einer Messfaser (8, 9) in das Schlauchlumen (2, 3) eingesetzt wird,
- wobei die Messfaser (8, 9) in Richtung einer Schlauch-Längsachse (14), also axial, länger ist als die zugehörige Fenster-Durchtrittsöffnung (11, 12; 18; 20; 21, 22).

8. Verfahren nach Anspruch 7, **gekennzeichnet durch** die Schritte:
- Entfernen eines Trennsteg-Abschnittes zwischen zwei der Schlauchlumen (2, 3) im Bereich des distalen Schlauchendes (5),
- Verschließen des distalen Schlauchendes (5).

9. Verfahren nach Anspruch 8, **dadurch gekennzeichnet, dass** das Verschließen durch Verkleben erfolgt.

## Claims

1. Multiple-lumen microcatheter tube (1)
- with at least two tube lumina (2, 3)
- with a closed distal tube end (5),
- wherein in the area of the distal tube end (5), a separation strip (4) comprises between the two tube lumina (2, 3) at least one passage opening (6),
- wherein at least one of the tube lumina (2, 3) comprises at least one window passage opening (11, 12; 18; 20; 21, 22), through which is made possible a medium exchange between the tube lumina (2, 3) and an environment (13),
- wherein in the area of the window passage opening (11, 12; 18; 20; 21, 22) is introduced a permeable material into the tube lumen (2, 3),
- **characterized in that** the permeable material is inserted into the tube lumen (2, 3) in the form of a measuring fiber (8, 9),
- wherein in the direction of a longitudinal axis (14) of the tube, i.e. axially, the measuring fiber (8, 9) is longer than the associated window passage opening (11, 12; 18; 20; 21, 22).

2. Microcatheter tube according to claim 1, **characterized in that** at least one of the tube lumina (2, 3) has a plurality of window passage openings (18; 20).

3. Microcatheter tube according to claim 1 or 2, **characterized in that** at least one of the tube lumina (2, 3) has exactly one window passage opening (11, 12; 21, 22).

4. Microcatheter tube according to any one of claims 1 to 3, **characterized by** an adhesive layer for fixing the measuring fiber (8, 9) in the tube lumen (2, 3).

5. Microcatheter tube according to any one of claims 1 to 4, **characterized in that** the distal tube end (5) is closed in a fluid-tight manner.

6. Microcatheter tube according to any one of claims 1 to 5, **characterized in that** the tube lumina (2, 3) have an inner diameter in the range between 0.1 mm and 2 mm.

7. Method for manufacturing a multiple-lumen microcatheter tube according to one of claims 1 to 6 with the following steps:
- producing a multiple-lumen tube,
- introduction of the at least one window passage opening (11, 12; 18; 20; 21, 22) into at least one of the tube lumina (2, 3),
- insertion of the permeable material (8, 9) into the tube lumen (2, 3) comprising the window passage opening (11, 12; 18; 20; 21, 22) at the height of the window passage opening (11, 12; 18; 20; 21, 22),
- fixing the permeable material in the tube lumina (2, 3) by gluing,
- **characterized in that** the permeable material is inserted into the tube lumen (2, 3) in the form of a measuring fiber (8, 9),
- wherein in the direction of a longitudinal axis (14) of the tube, i.e. axially, the measuring fiber (8, 9) is longer than the associated window passage opening (11, 12; 18; 20; 21, 22).

8. Method according to claim 7, **characterized by** the following steps:
- removing a separation strip section between two of the tube lumina (2, 3) in the area of the distal tube end (5),
- closing the distal tube end (5).

9. Method according to claim 8, **characterized in that** the closing occurs by gluing.

## Revendications

1. Tube de micro-cathéter à lumières multiples (1), comportant
- au moins deux lumières de tube (2, 3),
- une extrémité de tube distale fermée (5),
- dans lequel, dans la zone de l'extrémité de tube distale (5), une barrette de séparation entre les deux lumières de tube (2, 3) (4) comporte au moins une ouverture de passage (6),
- dans lequel au moins l'une des lumières de tube (2, 3) comporte au moins une ouverture de passage de fenêtre (11, 12 ; 18 ; 20 ; 21, 22), permettant un échange de support entre la lumière de tube (2, 3) et un environnement (13),
- dans lequel, dans la zone de l'ouverture de passage de fenêtre (11, 12 ; 18 ; 20 ;
21, 22), un matériau perméable est disposé dans la lumière de tube (2, 3),
- **caractérisé en ce que** le matériau perméable est utilisé comme matériau fibreux sous la forme d'une fibre de mesure (8, 9) dans la lumière de tube (2, 3),
- dans lequel la fibre de mesure (8, 9) dans le sens d'un axe longitudinal de tube (14), qui est par conséquent axialement plus long que l'ouverture de passage de fenêtre (11, 12 ; 18 ; 20 ; 21, 22) correspondante.

2. Tube de micro-cathéter selon la revendication 1, **caractérisé en ce qu'**au moins l'une des lumières de tube (2, 3) comporte une pluralité d'ouvertures de passage de fenêtre (18 ; 20).

3. Tube de micro-cathéter selon la revendication 1 ou 2, **caractérisé en ce qu'**au moins l'une des lumières de tube (2, 3) comporte exactement une ouverture de passage de fenêtre (11, 12 ; 21, 22).

4. Tube de micro-cathéter selon l'une des revendications 1 à 3, **caractérisé par** une couche adhésive pour la fixation de la fibre de mesure (8, 9) dans la lumière de tube (2, 3).

5. Tube de micro-cathéter selon l'une des revendications 1 à 4, **caractérisé en ce que** l'extrémité de tube distale (5) est étanche aux fluides.

6. Tube de micro-cathéter selon l'une des revendications 1 à 5, **caractérisé en ce que** les lumières de tube (2, 3) ont un diamètre interne compris entre 0,1 et 2 mm.

7. Procédé de fabrication d'un tube de micro-cathéter à lumières multiples selon l'une des revendications 1 à 6, comprenant les étapes :
- de fabrication d'un tube à lumières multiples,
- d'incorporation d'au moins une ouverture de passage de fenêtre (11, 12 ;18 ; 20 ; 21, 22) dans au moins l'une des lumières de tube (2, 3),
- d'incorporation du matériau perméable dans la lumière de tube (2, 3) comportant l'ouverture de passage de fenêtre (11, 12 ; 18 ; 20 ; 21, 22) au niveau de l'ouverture de passage de fenêtre (11, 12 ; 18 ; 20 ; 21, 22),
- de fixation du matériau perméable dans la lumière de tube (2, 3) par collage,
- **caractérisé en ce que** le matériau perméable est utilisé comme matériau fibreux sous la forme d'une fibre de mesure (8, 9) dans la lumière de tube (2, 3),
- dans lequel la fibre de mesure (8, 9) dans le sens d'un axe longitudinal de tube (14), qui est par conséquent axialement plus long que l'ouverture de passage de fenêtre (11, 12 ; 18 ; 20 ; 21, 22) correspondante.

8. Procédé selon la revendication 7, **caractérisé par** les étapes :
- de retrait d'une section de barrette de séparation entre deux des lumières de tube (2, 3) dans la zone de l'extrémité de tube distale (5),
- de fermeture de l'extrémité de tube distale (5).

9. Procédé selon la revendication 8, **caractérisé en ce que** la fermeture est réalisée par collage.
